# EUROPEAN PATENT SPECIFICATION

(11) **EP 3 590 915 B1**
(45) Date of publication and mention of the grant of the patent: **25.11.2020**
(21) Application number: 19460023.5
(22) Date of filing: 24.04.2019
(51) Int. Cl.: C07C 67/03, C07C 69/82, C08G 63/672, C08G 65/332

(54) **THE METHOD OF PREPARATION OF SURFACE-ACTIVE COMPOUNDS AND HIGH MOLECULAR WEIGHT PLASTICIZERS**
VERFAHREN ZUR HERSTELLUNG VON OBERFLÄCHENAKTIVEN VERBINDUNGEN UND HOCHMOLEKULAREN WEICHMACHERN
PROCÉDÉ DE PRÉPARATION DE COMPOSÉS ACTIFS EN SURFACE ET PLASTIFIANTS À POIDS MOLÉCULAIRE ÉLEVÉ

(30) Priority: 27.04.2018 PL 42539818
(43) Date of publication of application: 08.01.2020
(73) Proprietor: Purinowa spólka z o.o., 85-825 Bydgoszcz (PL)
(72) Inventor: Pienkowska, Marta, 85-825 Bydgoszcz (PL); Gawel, Grzegorz, 85-162 Bydgoszcz (PL); Korzeniowski, Krzysztof, 00-448 Warszawa (PL); Korzeniowski, Lukasz, 33-111 Koszyce MALE (PL)
(74) Representative: Karczmitowicz, Teresa Ewa

(56) References cited:
- EP-A1- 1 308 208
- US-A- 5 364 956

## Description

The subject matter of the invention is the method of obtaining high molecular mass plasticizers and surfactants and the resulting high molecular mass plasticizers and surfactants used in the field of plastics processing, emulsion polymerization and as emulsifiers, dispersants, viscosity modifiers, detergents and as additives to paints, varnishes, adhesives and binders.

The compounds with plasticizing and surfactant properties, belonging to several groups of chemical compounds are available on the market. The compounds based on phthalates, mainly orthophthalates - where the base substrate is phthalic anhydride (DOP, DBP, DIBP DINP, DIDP, DPHP) are popular and widely used in the field of plasticizers. Due to their structure, these compounds are well suited as plasticizers in the processing of polyvinyl chloride. In addition to phthalic anhydride based compounds, there are also other aromatic and aliphatic compounds, such as terephthalates (DEHT, DBT), adipinates (DEHA, DINA), benzoates, citrates, cyclohexanates.

The surfactants on the market are divided into ionic (cationic, anionic, amphoteric) and non-ionic compounds. Cationic surfactants are represented by amine salts, while anionic surfactants are represented by sodium and potassium salts, sulphates and nitrates. Amphoteric surfactants contain positive and negative ions in the same molecule (e.g. Sulfobetaine). Alkoxylates of acids, carboxylic alcohols, esters, amines and amides as well as vegetable oils are widely used among non-ionic surfactants. The derivatives of sucrose, lactose and sorbitol should also be mentioned.

Non-ionic polymerizable surfactants, derivatives of oxyethylated alcohols of polyoxyethylated fatty alcohols type, consisting of a saturated hydrocarbon chain of 4 to 18 carbon atoms and 2 to 23 oxyethylene groups, are known from the description of Polish patent No PL210339. The method of production of new non-ionic surfactants based on one-step reaction of polyoxyethylated fatty alcohol with simple, saturated bicarbon chain C4-C14 and 2-23 oxyethylene groups with 2.4-hexadiene chloride in polar solvent environment, preferably dichloromethane, at temperature up to 10°C is also known. The solvent is removed from the reaction mixture and the resulting product is purified by generally accepted methods. The main benefits of the invention lie in the possibility to introduce in a simple way a reactive sorbic grouping containing a coupled double bond system into the structure of a surfactant. Patent No PL189028 reveals how polyester plasticisers are manufactured by polycondensation of dicarboxylic acid, in particular adipic acid, and/or acid anhydride, in particular phthalic anhydride, with glycol or glycol mixture and esterification of the final groups with monocarboxylic acid or monohydroxy alcohol, with condensation water secretion and removal, where the adipic acid, phthalic anhydride or their mixture is subjected to polycondensation with glycol of the general formula HO-CH2- CR1R2-CH2-OH in which R1 and R2 are different and - if R1 denotes a methyl group, then R2 means the hydrogen atom or ethyl group and if R1 means the ethyl group, R2 means the n-butyl group or R1 and R2 together means the ring group (CH2)5 and together with the central carbon atom in the general formula HO-CH2-CR1R2-CH2-CH2-OH form the cyclohexane ring, or is subjected to polycondensation with the glycol mixture with the general formula HO-CH2- CR1R2-CH2-OH, in which R1 and R2 have the above meanings, with ethylene glycol, diethylene, neopentyl, 1,4-butylene, 1,2-propylene or 1,3-propylene glycol, where the content of glycol with the general formula HO-CH2CR1R2-CH2-OH in the glycol mixture is at least 5% molar and the end groups of the resulting product are esterified prefereably with 2-ethylhexanol.

Patent application US2018022891 (A1) entitled 'Mixture of plasticizers containing polymeric esters of dicarboxylic acids and dialkyl esters of terephthalic acid' revealed a plasticiser composition which contains at least one polymeric ester of dicarboxylic acid and at least one dialkyl terephthalate and the composition contains at least one compound with the general formula (I): 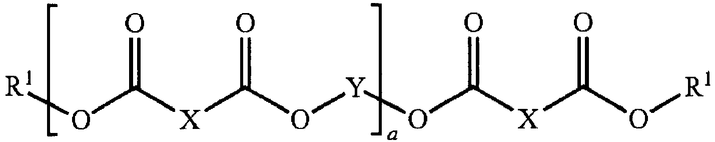 where:
X- is an unbranched or branched C2-C8 alkylene group or unbranched or branched C2-C8 alkylene group containing at least one double bond,
Y - means an unbranched or branched alkylene group C2-C12 or an unbranched or branched alkylene group C2-C12 alkylene containing at least one double bond,
a - is an integer from 2 to 50,
R 1 is classified as C4-C12 unbranched or branched alkyl radicals,
I At least one compound with general formula (II) 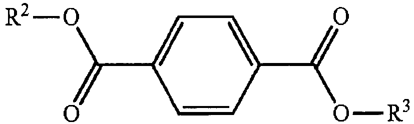 where:
   **R2** and **R3** belong to branched and unbranched C4-C12 alkyl radicals independently of each other.

This invention also revealed a composition containing at least one polymer and a plasticiser composition as defined above, in which the polymer is a thermoplastic polymer selected from among homopolymers or copolymers containing in polymerised form at least one monomer selected from C2-C10 monolefins, 1,3-butadiene, 2-chloro-1,3-butadiene, vinyl alcohol and its alkyl esters C2-C10, vinyl chloride, vinylidene chloride, vinylidene fluoride, tetrafluoroethylene, glycidyl acrylate, glycidyl methacrylate, acrylates and methacrylates of C1-C10 alcohols, vinyl aromas, (meth) acrylonitrile, maleic anhydride and α, β-ethylene unsaturated monocarboxylic and dicarboxylic acids, homopolymers and vinyl acetal copolymers, polyvinyl esters, polycarbonates, polyesters, polyethers, polyether ketones, thermoplastic polyurethanes, polysulphides, polysulphones, polyetherosulphones, cellulose alkyl esters and mixtures thereof.

The description of the international patent application No. WO2016/069673A1, entitled 'Polymer Plasticiser' revealed the composition of the plasticiser made on the basis of aromatic acid, glycol and C4-C36 monocarboxylic acid, their esters or anhydrides. Aromatic acid may be based on polymeric material such as recycled polyethylene terephthalate (PET). This invention also concerns the methods of the production of polymeric compositions of plasticisers, the methods of plasticisation of polymeric materials and the plasticised polymeric compositions. The method of production of a polymeric composition of a plasticizer consisting of the reaction of: aromatic acid, glycol and monocarboxylic acid C4-C36, its ester or anhydride at a temperature between 80° C and 260° C in the presence of a catalyst chosen from titanium catalysts, tin catalysts and their combinations. The variants of the production method of polymeric composition of the plasticizer also include: subjecting C4-C36 alcohol to the reaction; subjecting saturated or unsaturated aliphatic, linear, branched or cyclic aliphatic polyac acid or hydroxy substituted polyamide, or its esters or anhydrides to the reaction; subjecting hydroxyl substituted C3-C36 monocarboxylic acid, its ester or anhydride, and their combinations to the reaction.

The plasticisers according to the invention described are useful for plasticising various polymers, such as thermoplastic polymers, including for example polyvinyl chloride (PVC). Polymeric plasticisers are an alternative to conventional ones such as phthalic esters such as diisoctyl phthalate (DOP).

The next patent description PL/EP1912929 revealed the process of obtaining di-(2-ethylhexyl) terephthalate, which involves contact of terephthalic acid (TPA) with 2-ethylhexanol (EH) in the presence of a titanium catalyst in the reaction zone with a temperature of 180° to 270° C, molar ratio EH:TPA is in the range of 2:1 to 2,5:1 with total pressure in the reaction zone between 100 and 400 kPa (manometer 1 to 4 barg) and an inert gas is passed through the TPA/EH reaction mixture in the reaction zone to remove the water-EH mixture. Titanium tetraalcoholate with formula Ti (OR) 4 is used as titanium catalyst, R being an alkyl group with 1 to 8 carbon atoms per molecule and the concentration of the catalyst in the reaction mixture is the amount that provides a concentration of [Ti] between 50 and 200 parts per million by weight.

US5364956A discloses the preparation of a polyester comprising polyethylene glycol, phthalate and methylhexyl moieties.

Constantly increasing safety requirements for the use of chemical substances in order to better protect the environment and human health, as well as, in particular, the REACH Regulation requirements related to the need to identify and the specific control of risks associated with manufactured substances and placed on the EU market have motivated the Inventor to formulate and solve an inventive problem by developing technologies for the manufacture of of new types of surfactants, including high molecular mass plasticisers that are not harmful to health and do not contain commonly used phthalates and therefore can be safely used on the market.

The purposes of the invention is therefore to produce high molecular mass plasticisers and surfactants and high molecular mass plasticisers and surfactants obtained by the invention,-which are safe to use and constitute a new group of compounds which are an alternative to the existing ones. This goal was achieved by developing an innovative method of obtaining surfactants with the use of a system of devices used to implement the method.

The subject of the invention is the method of obtaining surfactants and high molecular mass plasticizers using transesterification process and esterification process using organometallic catalyst, where the method of obtaining surface-active compounds is based on transesterification of 2-ethylhexyl terephthalate in the amount of 30% to 80% by mass and 20 to 70% by mass of polyethylene glycol of molecular weight from 200 to 600 g/mol and heating the reactor with activated mixing and nitrogen supply from 50 to 200 l/min to temperature from 160 to 200 °C and then introducing of liquid organometallic catalyst into the reaction environment in the amount from 0,02 to 2% by mass and conducting the process to obtain the assumed parameters in accordance with the mass balance, simultaneously discharging the resulting 2-ethylhexyl alcohol to the reactor and finishing the process by rapid cooling of the finished product remaining in the reactor to the temperatures from 80 to 120°C and the elimination of negative pressure, while the method of obtaining high molecular mass plasticizers is based on the fact that the esterification process is carried out by introducing 2-ethylhexyl alcohol and alcohols with the content of carbon atoms in the C2-C4 molecule and acids with the content of carbons in the C4-C6 molecule with a mass ratio of acid substrates of 25 to 60% by mass and hydroxy substrates containing hydroxyl group of 40 to 75% by mass, wherein among all hydroxyl substrates the 10 to 80% by mass is constituted by 2-ethylhexyl alcohol and from 20 to 90% by mass are other hydroxyl substrates, then the reactor content is heated to the temperature from 150 to 180°C under the absolute pressure from 30 to 500 mmHg (4.0 to 66.7 kPa) and an organometallic catalyst is dosed successively in the amount from 0.02 to 2% by mass and the process is carried out to obtain the parameters of the acid number below 1 mgKOH/100g and viscosity within the range from 15 to 1000 mPas, while draining the by-products in the form of water and 2-ethylhexyl alcohol, then the process ends by cooling the product to the temperature from 80 to 120°C and removing the vacuum.

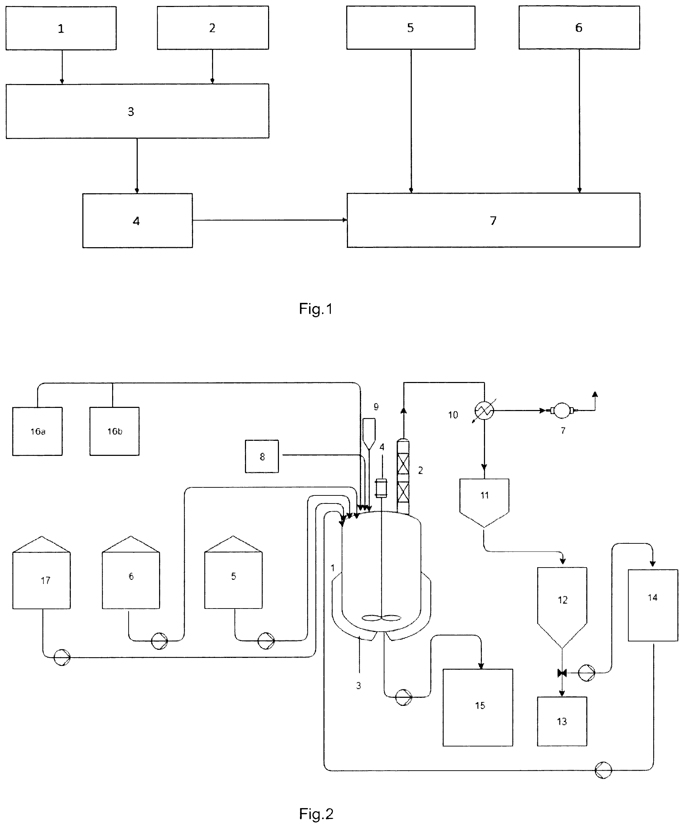

The method according to the invention is presented in the process diagram for obtaining plasticizers and surfactants, in the drawing - Fig. 1.

The method of obtaining surfactants by transesterification according to the invention consists in introducing 2-ethylhexyl terephthalate into the reactor in the amount from 30% to 80% by mass and ethylene polyglycol with the molecular weight of 200 to 600 g/mol in the amount from 20% to 70% by mass. The reactor content is heated to the temperature from 150 to 200°C with mixing switched on at the speed from 50 to 1000 rpm and nitrogen supply from 50 to 200 l/min. Then a liquid catalyst is introduced into the reaction environment in the amount from 0.02 to 2% by mass, with the introduction of the catalyst taking place at the temperature not higher than 180°C. During the transesterification reaction, 2-ethylhexyl alcohol is produced as a by-product in the amount of 15% to 35% in relation to the weight of the main product, i.e. 2-etulohexyl teraphthalate. The 2-ethylhexyl alcohol is discharged from the reaction environment through a distillation column and a cooler to the distillate receiver, from where it is transported to the 2-ethylhexanol tank.

In this way, the separated by-product of the transesterification reaction, i.e. 2-ethylhexyl alcohol, can be used successively as a substrate in the esterification process, especially since 2-ethylhexyl alcohol contains up to 0.1% water and can be successfully used to obtain aliphatic plasticizers or can be an excellent raw material for use in other production processes.

The rate of removal of 2-ethylhexyl alcohol from the reaction environment is regulated by temperature in the range from 150 to 200°C and pressure from 30 to 500 mmHg (4.0 to 66.7 kPa), preferably from 50 to 80 mmHg (6.7 to 10.7 kPa). During the reaction the following product parameters are controlled: viscosity, acid and hydroxyl numbers, as well as the quantity of 2-ethylhexanol discharged from the reaction environment. The end of the reaction after obtaining the assumed parameters and receiving the quantity of 2-ethylhexanol according to the mass balance of the reaction is achieved by rapid cooling of the product in the reactor to the temperature from 80 to 120°C at an absolute pressure from 30 to 500 mmHg (4.0 to 66.7 kPa), preferably from 50 to 80 mmHg (6.7 to 10.7 kPa).

In the transesterification process there is a reaction between 2-ethylhexyl terephthalate and polyethylene glycol in the presence of an organometallic catalyst, which results in aromatic polyetheropolyesters - polyethylene glycol terephthalates.

**(1)** R1 - COO - R2 - COO- R1 + H(OCH2CH2)nOH ↔ R1 - COO - R2 - COO-[CH2CH2O]nH + R10H,

**(2)** mR1 - COO - R2 - COO- [CH2CH2O]nH + H(OCH2CH2)nOH ↔ H(OCH2CH2)n-O-{CO - R2 - COO-[CH2CH2O]n}m H + m R1OH

**(3)** H(OCH2CH2)n - O -{CO - R2 - COO- [CH2CH2O]n}m H + R1 - COO - R2-COO R1 ↔ R1 - COO - R2 - COO-{(CH2CH2O)n - CO - R2 - COO}m-[CH2CH2O]nH + R1OH,

where:
R1 - stands for 2-ethylhexyl alkyl chain with the molecular formula C8H17;
R1OH - means 2-ethylhexyl alcohol with the molecular formula C8H18O;
R2 - means a benzene ring substituted in the *para*- position (1,4), with the molecular formula C6H4;
R1 - COO - R2 - COO - R1 - means dioctyl terephthalate with the molecular formula C24H38O4;
H(OCH2CH2)nOH - stands for polyethylene glycol,
*n>4 and m≥1.*

The reactions described above by general equations (1), (2), (3) occur in a mixture of substrates, i.e. dioctyl teraphthalate and polyethylene glycol, depending on the proportion of these substrates. The basic reaction is the transesterification reaction of diisoctyl terephthalate with polyglycol, described by equation (1).

In the presence of a molar excess of polyglycol further reactions of attaching the polyglycol to the second carboxylate group of diisooctyl terephthalate are taking place, described by the general equation (2) and successive molecules joining one another, described by the general equation (3).

Polyglycol terephthalates, i.e. surfactants with plasticizing properties, are produced as a result of transesterification reaction.

In the process of transesterification by the method according to the invention, the compounds with different parameters of average molecular weight from 1000 to 1700 g/mol and the coefficient HLB 9-17 are obtained in the reaction of dioctyl terephthalate transesterification by quantitative change of raw material charge of dioctyl terephthalate in the range from 30% to 80% by mass and ethylene polyglycol in the range from 20 to 70% by mass or by using ethylene polyglycol of different molecular weight in the range from 200 to 600 g/mol.

Surfactants with plasticizing properties, manufactured according to equation (1), have a molecular weight between 450 and 900 g/mol and are determined by the general formula **(4)** R1 - COO - R2 - COO - [CH2CH2O]nH ,
Where:
R1 - stands for 2-ethylhexyl alkyl chain with the molecular formula C8H17,
R2 - means a benzene ring substituted in the *para-* position with the molecular formula C6H4 and n> 4.
Plasticizing surfactants manufactured according to equation (2) have a molecular weight between 500 and 1400 g/mol and are determined by the general formula (5) H(OCH2CH2)n - O -{CO - R2 - COO- [CH2CH2O]n}m H,
where:
R2 - means a benzene ring substituted in the *para-* position with the molecular formula C6H4, and n> 4.
Plasticizing surfactants manufactured according to equation have a molecular weight between 750 and 2350 g/mol and are determined by the general formula **(6)** R1 - COO - R2 - COO-{(CH2CH2O)n - CO - R2 - COO}m - [CH2CH2O]nH,
Where:
R1 - stands for 2-ethylhexyl alkyl chain with the molecular formula C8H17,
R2 - means a benzene ring substituted in the *para-* position with the molecular formula C6H, n>4 and m≥1.

The method according to the invention, produces a significant amount of by-product in the form of 2-ethylhexyl alcohol, which, if taken away during the transesterification process, allows its use it in the synthesis of other compounds. 2-ethylhexyl alcohol received during the transesterification process constitutes from 15% to 35% of the weight of the main product.
The assumption of the transesterification process described by equations (1) to (3) is to conduct it towards the total transesterification of the substrate and the creation of structures described by these equations by selecting the type and molar proportions of the components. and selection of the process conditions, which results in different molar masses and HLB coefficients selected for a given (specific) application.

Another process of the method according to the invention is the esterification process to which the following substrates are subjected: glycol with two to four carbon atoms in a molecule, acids with four to six carbon atoms in in a molecule and 2-ethylhexanol as a by-product of the transesterification reaction.
2-Ethylhexyl alcohol, alcohols with carbon content in the C2-C4 molecule and acids with carbon content in the C4-C6 molecule are introduced into the reactor, where the content of acidic substrates is from 25 to 60% by mass and the content of hydroxy substrates containing hydroxyl group is from 40 to 75% by mass, whereas among all hydroxy substrates from 10 to 80% by mass are 2-ethylhexyl alcohol and from 20 to 90% by mass are other hydroxy substrates.
Then the reactor content is heated to the temperature from 150 to 200°C, at the absolute pressure from 30 to 500 mmHg (4.0 to 66.7 kPa), preferably from 50 to 80 mmHg (6.7 to 10.7 kPa), and then the catalyst content is dosed in the amount from 0.02 to 2% by mass, while the temperature of catalyst dosing may not exceed 180°C.
During the esterification process, water is released as a by-product, which, due to its maintained temperature, evaporates with a significant amount of 2-ethylhexanol, which accounts for up to 70% of the distillate by mass, and this vapour mixture is discharged through a distillation column and a cooler in which it is condensed into the distillate receiver. The contents of the receiver are then transferred to a tank in which the phase separation between process water and 2-ethylhexanol takes place. The resulting 2-ethylhexanol is returned to the reaction environment or used for other syntheses if the reaction environment is supplemented with fresh 2-ethylhexanol. After obtaining acid number parameters below 1 mgKOH/100g, viscosity within 15-1000 mPas, as well as removing the residue of 2-ethylhexyl alcohol, the process is completed by cooling the product to the temperature from 80 to 120 °C and removing the negative pressure.

In result of the process of esterification of glycols, acids and in the presence of excess 2-ethylhexanol with the use of organometallic catalysts a number of polymeric products with general equations (7), (8) and (9) is produced.

**(7)** (n+1) OH-R3-OH + (n+1) COOH-R4-COOH + R1-OH -> OH-R3-COO-R4-COO-[R1-COO-R4-COO]n-R1 + (n+2)H2O,

**(8)** HOOC - R4 - COOH + HO-R3-OH + 2 R1-OH -> OH-R3-COO-R4-CO-O-R3-COO-R4-COO-R1 + 2H2O

**(9)** n HO-R3-OH + (n+1) HOOC-R4-COOH + (n+1) R1OH -> R1-COO-R4-COO-[R3-COO-R4-COO]n-R1 + (n+3) H2O,

where:
R1 - stands for alkyl chain of 2-ethylhexyl alcohol with the molecular formula C8H17;
R3 stands for aliphatic alkyl chain with a number of carbon atoms 2-4 (C2-C4);
R4 stands for aliphatic alkyl chain a number of carbon atoms 4-6 (C4-C6),
and n ≥ 1

The esterification process results in aliphatic polyesters and polyether-polyesters. with strong plasticizing properties and at the same time acting as viscosity regulators.

Aliphatic polyesters and polyether-polyesters are formed in the reaction of esterification of dicarboxylic acids, polyols and polyethylene glycols, wherein the percentage by mass of acidic substrates is between 25 and 60% and hydroxy substrates containing hydroxyl group is between 40 and 75%.

The resulting polyester is characterized by an average molecular weight from 500 to 2000 g/mol, in which the carboxylic groups of the resulting polyester are esterified with 2-ethylhexyl alcohol remaining in excess in the reaction environment. The carboxyl groups of polyester can also react with the polyglycol to form a polyester with a mass of 700 to 3500 g/mol.

Plasticisers manufactured according to equation (7) have a molecular weight between 450 and 2000 g/mol and are determined by the general formula (10) OH-R3-COO-R4-COO-[R1-COO-R4-COO]n-R1 + (n+2)H2O,
where:
R1 - 2-ethylhexyl alcohol alkyl chain,
R3 stands for aliphatic alkyl chain a number of carbon atoms 2-4 (C2-C4),
R4 stands for aliphatic alkyl chain with a number of carbon atoms 4-6 (C4-C6);
and n ≥ 1
Plasticisers manufactured according to equation (8) have a molecular weight between 550 and 650 g/mol and are determined by the general formula (11) OH-R3-COO-R4-CO-O-R3-COO-R4-COO-R1,
Where:
R1 - stands for alkyl chain of 2-ethylhexyl alcohol;
R3 stands for aliphatic alkyl chain a number of carbon atoms 2-4 (C2-C4),
R4 stands for aliphatic alkyl chain a number of carbon atoms 4-6 (C4-C6),
and n ≥ 1
Plasticisers manufactured according to equation (9) have a molecular weight between 700 and 850 g/mol and are determined by the general formula (12) R1-COO-R4-COO-[R3-COO-R4-COO]n-R1,
where:
R1 - stands for alkyl chain of 2-ethylhexyl alcohol;
R3 stands for aliphatic alkyl chain with a number of carbon atoms 2-4 (C2-C4);
R4 stands for aliphatic alkyl chain a number of carbon atoms 4-6 (C4-C6),
and n ≥ 1
Surfactants and plasticizing agents, obtained by the method according to the the invention, constitute a new group of compounds constituting an alternative to the ones used so far. They are distinguished by a high molecular weight structure, and depending on the molecular weight and the way of conducting the reaction, their hydro-lipophilic properties can be regulated in a wide range. Moreover, surfactants and plasticizers are not classified as hazardous compounds according to the current regulations.

The method according to the invention is based on the processes of transesterification and esterification with the use of organometallic catalysts and the use of alcohol produced in the transesterification reaction. The added value of the method according to the invention is that the processes are carried out periodically, using a reactor equipped with devices enabling both heating and cooling of its content, a mixer, an inert gas supply and a distillation column with a distribution capacity equivalent to at least 5 theoretical plates.
The absolute pressure range during conducting the transesterification and esterification processes is between 30 and 500 mmHg (4.0 to 66.7 kPa), preferably from 50 to 80 mmHg (6.7 to 10.7 kPa), and the temperature range of the transesterification and esterification processes is between 150 and 200 °C.

Titanium, zirconium or tin compounds, preferably tetra-n-butyl titanate or tetra-n-butyl zirconate, tin octanoate, dibutyltin oxide or dioctyltin oxide are used as organometallic catalysts. Catalysts shall be introduced directly into the reaction environment in the quantity of 0,02 to 2,0 % by mass.

The compounds, obtained by the method according to the invention, are polyether-polyesters, which are characterized by the presence of an ester bond and a polyether chain in the molecule and their polymeric structure is conditioned by the presence of n>4 repetitive units of ethylene oxide from polyglycol or n>4 repetitive units of ester monomer, formed by the esterification reaction of polyols and dicarboxylic acid.

The method according to the invention allows to obtain a wide range of compounds differing in physicochemical properties and surface-active properties, which significantly broadens their applicability. The method according to the invention ensures optimal use of by-products arising in the processes.

The process of transesterification and esterification according to the invention is carried out in a system of devices, the diagram of which is shown in Fig. 2.

The system of devices enabling the implementation of the invention consists of a reactor equipped with a distillation column with a distribution capacity equivalent to at least 5 theoretical plates, a heating and cooling jacket, a cooler, a vacuum pump, a mechanical stirrer and a set of manipulation tanks configured so that two separate processes of transesterification and esterification can be carried out consecutively in one reactor.

As shown in Fig. 2, the transesterification reaction is carried out by dosing 2-ethylhexyl terephthalate from tank 5 and polyethylene glycol from tank 6, respectively, into reactor 1 with distillation column 2, heating and cooling jacket 3 and agitator 4. The content of the reactor 1 is heated to the temperature from 150 to 2000°C with the vacuum pump 7 switched on, agitator 4 switched on at the mixing speed of 50 to 1000 rpm, then nitrogen from the container 8 and catalyst from the container 9 is dosed into the reactor 1. After the process is complete, the distillate (2-ethylhexanol and water) is discharged through the column 2 and the radiator 10 to the distillate receiver 11.

The distillate from the receiver 11 is transferred to the retention tank 12, where due to the difference in density, the bottom layer, i.e. water, separates from 2-ethylhexanol and is discharged to the tank 13, while the top layer (2-ethylhexanol) is pumped to the tank 14.

The finished product, namely, polyglycol terephthalate is pumped from the reactor 1 to the tank 15.

The esterification reaction is carried out by pumping 2-ethylhexanol from the tank 14 to the reactor 1 and carboxylic acids C4-C6 from tanks 16a and 16b and alcohol C2-C4 from the tank 17. Heating, mixing, nitrogen supply from the tank 8 is started and the vacuum pump 7 is activated in the reactor 1 and then the catalyst is dosed from tank 9.

The distillate (2-ethylhexanol and water) is discharged through column 2 of the cooler 10 to the receiver 11.

The distillate is transferred from the receiver 11 to the tank 12 where, due to the difference in density, the water generated in the process is separated from the 2-ethylhexanol. The lower layer (process water with residual raw materials) is discharged to the tank 13, while the upper layer (2-ethylhexanol) is pumped to the tank 14 from where it can be returned to the process. The finished product is pumped from the reactor 1 to the tank 15.

The method according to the invention is presented in the embodiments

### Example 1.

The reactor was dosed with 1610 kg of dioctyl terephthalate and 1692 kg of polyethylene glycol. The stirrer, nitrogen supply and heating were switched on. When the substrate mixture reached 160°C, a catalyst, i.e. tin octanoate, was added in the quantity of 1.35 kg, and the reactor content was heated to 175°C. Under these conditions the reaction was carried out in result of which 2700 kg of product was obtained with viscosity of 435 mPas at 25°C, acid number below 0.2 mgKOH/g and 535 kg of 2-ethylhexanol, which during the reaction was stripped and distilled in the distillation column and directed to the distillate receiver. The 2-ethylhexanol obtained in this way has a purity of 98.5% and max. water content 0.1% m/m, and can be used as a raw material for further syntheses. The obtained ester of terephthalic acid and polyglycol is a surfactant with HLB 13,4 and is used as a detergent for surface cleaning, machine washing in dishwashers and in formulation of polyurethane systems for obtaining rigid foam. It can also be used as a plasticizer, e.g. in the processing of polyvinyl chloride.

### Example 2.

The reactor was fed with 1950 kg dioctyl terephthalate and 750 kg polyethylene glycol. Nitrogen supply, mixing and gradual heating to 165°C were switched on, after which 1.6 g of tin catalyst was added. The reaction was carried out at 165°C, controlling the acid number and viscosity of the mixture. 2000 kg of product was obtained with viscosity of 248 mPas and hydroxyl number 45 mgKOH/g, and 640 kg of 2-ethylhexanol, which after discharge to the receiver and further to the tank of 2-ethylhexanol contains a maximum of 0.1% water and minimum of 98.5% ethylhexanol, and can be used as a raw material for further syntheses. The obtained polyether polyester is a surfactant with HLB 16, used in washing detergents and polishing agents.

### Example 3.

The reactor was fed with 1950kg dioctyl terephthalate and 1500kg polyethylene glycol. Heating, mixing and nitrogen supply were switched on. After reaching 160°C, a catalyst - dibutyltin dilaurate in the amount of 2.3kg - was added. The process was then carried out at 180-200°C. The product of viscosity 5339 mPas and acid number 0.84 mgKOH/g were obtained in the quantity of 1980 kg. 560 kg of the distillate was discharged to the distillate receiver, from where it is transported to the 2-ethylhexanol tank. In this way, the separated 2-ethylhexyl alcohol contains up to 0.1% water. The HLB 9 polyester obtained by synthesis can be used as a surfactant in detergents and as a component of polyurethane systems.

### Example 4.

80 kg of 2-ethylhexyl alcohol from isooctyl terephthalate transesterification, 115 kg of monoethylene glycol and 118 kg of succinic acid and 146 kg of adipic acid were introduced into the reactor. Mixing, heating, and nitrogen supply were switched on. After reaching a constant temperature of 180°C tetrabutyl titanate catalyst was added. After two hours, 120 kg of 2-ethylhexyl alcohol was added to the reactor. After three hours the reaction temperature was reduced to 160°C and the nitrogen flow increased to remove excess of unreacted 2-ethylhexyl alcohol. 260 kg of distillate was removed from the receiver, which after transport from the receiver was separated from the receiver, resulting in 180 kg of 2-ethylhexyl alcohol for further use and 80 kg of water for disposal. A 400 kg product with a viscosity of 36 mPas, an acid number of 0.11 mg KOH/l and a molecular weight of about 700 g/mol was obtained for use as a plasticizer in the processing of polyvinyl chloride.

### Example 5.

1565 kg of 2-ethylhexanol (from synthesis with dioctyl terephthalate), 705 kg adipic acid, 570 kg succinic acid and 970 kg polyglycol were introduced into the reactor. Nitrogen flow, mixing and heating were switched on. A catalyst was added at 165°C: 0.72 kg Tetrabutyl titanate and 0.36 kg Tin oxalate. The reaction was carried out at the temperature of 195°C. 1050 kg of distillate containing 315 kg of water and 735 kg of 2-ethylhexyl alcohol were discharged to the receiver. 2,600 kg of aliphatic polyether with viscosity of 96 mPas and acid number of 0.46 mgKOH/g were obtained, which can serve as a plasticizer and a viscosity reducer.

### Example 6.

465 kg of succinic acid, 575 kg of adipic acid, 453 kg of monoethylene glycol and 788 kg of 2-ethylhexanol were introduced into the reactor. Nitrogen flow, mixing and heating were switched on. After reaching 180°C, a catalyst of tetrabutyl titanate in the amount of 1.13 kg was dosed. The reaction was continued at 180 - 200°C. 615 kg of distillate containing 296 kg of waste water and 315 kg of 2-ethylhexyl alcohol were discharged to the receiver. 1500 kg of product with viscosity 390 mPas and acid number 0.24 mgKOH/g were obtained, which can serve as plasticizer and viscosity reducer.

## Claims

1. The method of preparation of surface-active compounds and high molecular weight plasticizers using the transesterification process in the presence of a metalorganic catalyst, **characterized in that** the transesterification reaction is carried out by introducing into the reactor di(2-ethylhexyl) terephthalate in the amount of 30% to 80% by mass and 20 to 70% by mass of ethylene polyglycol with molecular weight from 200 to 600 g/mol, and the reactor content is heated with the mixing switched on and the nitrogen supply from 50 to 200 l/min to 160 to 200 °C and then a liquid organometallic catalyst is introduced into the reaction environment in the amount of 0,02 to 2% by mass and the process is carried out to obtain the assumed parameters in accordance with the mass balance, transferring simultaneously to the reactor the resulting 2-ethylhexyl alcohol, after which the process is terminated by rapid cooling of the product remaining in the reactor ready to temperature from 80 to 120°C and elimination of negative pressure, while the transesterification process takes place in accordance with following reactions of general equations:
(1) R1 - COO - R2 - COO- R1 + H(OCH2CH2)nOH ↔ R1 - COO - R2 - COO-[CH2CH2O]ₙH + R1OH,
(2) m R1 - COO - R2 - COO- [CH2CH2O]ₙH + H(OCH2CH2)ₙOH ↔ H(OCH2CH2)ₙ - O -{CO - R2 - COO- [CH2CH2O]ₙ}ₘ H + m R1OH, oraz
(3) H(OCH2CH2)n - O - {CO - R2 - COO- [CH2CH2O]n}m H + R1 - COO - R2 - COO-R1 ↔ R1 - COO - R2 - COO-{(CH2CH2O)n - CO - R2 - COO}m - [CH2CH2O]nH + R1OH,
where:
R1 - stands for 2-ethylhexyl alkyl chain with the molecular formula C8H17;
R1OH - means 2-ethylhexyl alcohol with the molecular formula C8H180;
R2 is the benzene ring substituted in the *para*- position with the molecular formula C6H4;
R1 - COO - R2 - COO - R1 is the dioctyl terephthalate with the molecular formula C24H38O4; H(OCH2CH2)nOH is the ethylene polyglycol, n>4 and m≥1.

2. The method according to claim 1 is **characterized in that** the transesterification process is carried out at absolute pressures of 4.0 to 66.7 kPa (30 to 500 mmHg), preferably from 6.7 to 10.7 kPa (50 to 80 mmHg).

3. The method according to claim 1 or 2 is **characterized in that** the transesterification process is carried out at the mixing speed from 50 to 1000 rpm.

4. The method according to any of the claims 1 to 3, **characterized in that** the transesterification process produces 2-ethylhexyl alcohol in the amount of 15% to 35% of the weight of the main product as a by-product.

5. The method according to any of the above claims 1 to 4, **characterized in that** the surfactants with plasticising properties produced in accordance with equation (1) have a molecular weight between 450 and 900 g/mol and are determined by the general formula (4) R1 - COO - R2 - COO - [CH2CH2O]ₙH , where R1 is the 2-ethylhexyl alkyl chain with the molecular formula C8H17, R2 is the benzene ring substituted in the *para-*position with the molecular formula C6H4 and n>4

6. The method according to any of the above claims 1 to 4 **characterized in that** the surfactants with plasticizing properties produced in accordance with equation (2) have a molecular weight from 500 to 1400 g/mol and are determined by the general formula (5) H(OCH2CH2)ₙ - O - {CO - R2 - COO- [CH2CH2O]ₙ}ₘ H, , where, R2 stands for a benzene ring substituted in the *para-* position with the molecular formula C6H4, and n>4.

7. The method according to any of the above claims 1 to 4 **characterized in that** the surfactants with plasticizing properties produced in accordance with equation have a molecular weight between 750 and 2350 g/mol and are determined by the general formula (6) R1 - COO - R2 - COO-{(CH2CH2O)n - CO - R2 - COO}m - [CH2CH2O]nH, where R1 is the 2-ethylhexyl alkyl chain with the molecular formula C8H17, R2 is the benzene ring substituted in the *para*- position with the molecular formula C6H4, n>4 and m≥1.

8. The method according to claim 1 further comprising the esterification process in the presence of an organometallic catalyst and 2-ethylhexyl alcohol formed in the transesterification process, **characterized in that** the esterification process is carried out with the introduction of 2-ethylhexyl alcohol and alcohols with a content of carbons in the molecule C-C4 and acids with a content of carbons in the molecule C4-C6 in mass ratio of acidic substrates of 25 to 60 % by weight and hydroxyl-containing substrates containing hydroxyl group ranging from 40 to 75% by mass, where 2-ethylhexyl alcohol constitutes 10 to 80% by mass of all hydroxyl-containing substrates and other hydroxyl-containing substrates constitutes from 20 to 90% by mass, after which the reactor content is heated to the temperature from 150 to 180°C at absolute pressure from 4.0 to 66.7 kPa (30 to 500 mmHg) and a metal-organic catalyst is dosed in amounts from 0,02 to 2% mass and the process is carried out to obtain acid number parameters below 1 mgKOH/100g and viscosity within the range of 15 to 1000 mPas, at the same time draining the by-products in the form of water and 2-ethylhexyl alcohol, after which the process is finished by cooling the product to the temperature of 80 to 120 °C and eliminating negative pressure, while the esterification process takes place according to the reactions of general equations:
(7) (n+1) OH-R3-OH + (n+1) COOH-R4-COOH + R1-OH -> OH-R3-COO-R4-COO-[R1-COO-R4-COO]n-R1 + (n+2)H2O,
(8) HOOC - R4 - COOH + HO-R3-OH + 2 R1-OH -> OH-R3-COO-R4-CO-O-R3-COO-R4-COO-R1 + 2H2O
(9) n HO-R3-OH + (n+1) HOOC-R4-COOH + (n+1) R1OH -> R1-COO-R4-COO-[R3-COO-R4-COO]n-R1 + (n+3) H2O
where:
R1 - stands for alkyl chain of 2-ethylhexyl alcohol with the molecular formula C8H17;
R3 stands for aliphatic alkyl chain with carbon number 2-4 (C2-C4);
R4 stands for aliphatic alkyl chain with carbon number 4-6 (C4-C6),
and n ≥ 1

9. The method described in claim 8 **characterized in that**, the esterification process is carried out at a pressure of 6.7 to 10.7 kPa (50 to 80 mmHg).

10. The method according to the claim 8 or 9 **characterized in that**, the distillate of by-products in the form of water and 2-ethylhexyl alcohol is transferred to the receiver and subsequently to the tank where the phase separation between process water and 2-ethylhexyl alcohol takes place, while the content of 2-ethylhexyl alcohol in the by-product is up to 70% by mass.

11. The method according to any of the above claims 8 to 10 **characterized in that**, the plasticisers produced according to equation (7) have a molecular weight of 450 to 2000 g/mol and are determined by the general formula (10) OH-R3-COO-R4-COO-[R1-COO-R4-COO]n-R1 + (n+2)H2O where:R1 - 2-ethylhexyl alcohol alkyl chain, R3 - aliphatic alkyl chain with a number of carbon atoms 2-4 (C2-C4), R4 - aliphatic alkyl chain with a number of carbon atoms 4-6 (C4-C6); and n ≥ 1,

12. The method according to any of the above claims 8 to 10, **characterized in that** the plasticisers produced according to equation (8) have a molecular weight of 550 to 650 g/mol and are determined by the general formula (11) OH-R3-COO-R4-CO-O-R3-COO-R4-COO-R1, where: R1 - 2-ethylhexyl alcohol alkyl chain, R3 - aliphatic alkyl chain with a number of carbon atoms 2-4 (C2-C4), R4 - aliphatic alkyl chain with a number of carbon atoms 4-6 (C4-C6); and n ≥ 1

13. The method according to any of the above claims 8 to 10, **characterized in that** the plasticisers produced according to equation (9) have a molecular weight between 700 and 850 g/mol and are determined by the general formula (12) R1-COO-R4-COO-[R3-COO-R4-COO]n-R1, where: R1 - 2-ethylhexyl alcohol alkyl chain, R3 - aliphatic alkyl chain with a number of carbon atoms 2-4 (C2-C4); R4 - aliphatic alkyl chain with a number of carbon atoms 4-6 (C4-C6), and n ≥ 1

14. The method according to any of the above claims from 1 to 13, **characterized in that** the catalyst dosing is carried out at the temperature not exceeding 180°C.

15. The method according to any of the above claims from 1 to 14 **characterized in that** titanium, zirconium or tin compounds are used as catalysts.

16. The method according to the claim 15 **characterized in that** the catalysts shall be tetra-n-butyl titanate, tetra-n-butyl zirconate, tin octanoate, dibutyltin oxide or dioctyltin oxide.

## Patentansprüche

1. Ein Verfahren, nach welchem oberflächenaktive Verbindungen und hochmolekulare Plastifiziermittel durch Umesterungsprozesses unter Zusatz eines metallorganischen Katalysators gewonnen werden, **gekennzeichnet dadurch, dass** die Umesterungsreaktion dadurch stattfindet, dass in den Reaktor Di(2-ethylhexyl)terephthalat in einer Menge von 30% bis 80% der Masse und von 20 bis 70% der Masse Poly(ethylen)glykol mit der Molmasse von 200 bis 600 g/mol eingeführt werden, und der Inhalt des Reaktors unter eingeschaltetem Mischen und Zuführung des Stickstoffes von 50 bis 200 I/Min bis 160 bis 200 °C angewärmt wird, wonach in das Reaktionsmedium ein flüssiger metallorganischer Katalysator in einer Menge von 0,02 bis 2% massenhaft eingeführt wird, und der Prozess zwecks Erzielung der angenommenen Parameter in Übereinstimmung mit der Massenbilanz geführt wird, wobei gleichzeitig der gewonnene 2-Ethylhexanol in den Rektor übertragen wird, wo anschließend der Prozess mit einer schnellen Abkühlung des fertigen im Reaktor verbleibenden Produktes bis auf die Temperatur von 80 bis 120°C und dem Ausschließen des Unterdruckes endet, und der Umesterungsprozess in Übereinstimmung mit folgenden Reaktionen nach den allgemeinen Gleichungen stattfindet:
(1) R1 - COO - R2 - COO- R1 + H(OCH2CH2)nOH ↔ R1 - COO - R2 - COO-[CH2CH2O]ₙH + R1OH,
(2) m R1 - COO - R2 - COO- [CH2CH2O]ₙH + H(OCH2CH2)ₙOH ↔ H(OCH2CH2)ₙ - O -{CO - R2 - COO- [CH2CH2O]ₙ}ₘ H + m R1OH,
sowie
(3) H(OCH2CH2)n - O - {CO - R2 - COO- [CH2CH2O]n}m H + R1 - COO - R2 - COO-R1 ↔ R1 - COO - R2 - COO-{(CH2CH2O)n - CO - R2 - COO}m - [CH2CH2O]nH + R1OH,
wo :
R1 - 2-Ethylhexylalkylkette mit der Molekularformel C8H17 bedeutet; R1OH - 2-Ethylhexanol mit der Molekularformel C8H18O bedeutet;
R2 ein an der geraden Position ersetzter Benzolring mit der Molekularformel C6H4 ist;
R1 - COO - R2 - COO - R1 Dioctylterephthalat mit der Molekularformel C24H38O4 ist; H(OCH2CH2)nOH ist Poly(ethylen)glykol, n>4 und m≥1.

2. Ein Verfahren gemäß Anspruch 1, **gekennzeichnet dadurch, dass** der Umesterungsprozess unter absolutes Drücken von 4,0 bis 66,7 kPa (30 do 500 mmHg), vorteilhaft von 6,7 bis 10,7 kPa (50 do 80 mmHg) durchgeführt wird.

3. Ein Verfahren gemäß Anspruch 1 oder 2, **gekennzeichnet dadurch, dass** der Umesterungsprozess mit der Mischgeschwindigkeit von 50 bis 1000 U/Min. durchgeführt wird.

4. Ein Verfahren gemäß einem beliebigen der Ansprüche von 1 bis 3, **gekennzeichnet dadurch, dass** im Umesterungsprozess 2-Ethylhexanol in einer Menge von 15% bis 35% der Masse des Hauptproduktes als Nebenprodukt entsteht.

5. Ein Verfahren gemäß einem beliebigen der Ansprüche von 1 bis 4, **gekennzeichnet dadurch, dass** Tenside mit plastifizierenden Eigenschaften, die in Übereinstimmung mit der Gleichung (1) produziert werden, eine Molmasse zwischen 450 und 900 g/mol haben und mit der allgemeinen Formel (4) R1 - COO - R2 - COO - [CH2CH2O]ₙH bezeichnet sind, wo R1 2-Ethylhexylalkylkette mit der Molekularformel C8H17, R2 ein an der geraden Position ersetzter Benzolring mit der Molekularformel C6H4 i n>4 sind.

6. Ein Verfahren gemäß einem der vorstehenden beliebigen Ansprüche 1 bis 4, **gekennzeichnet dadurch, dass** Tenside mit plastifizierenden Eigenschaften, die in Übereinstimmung mit der Gleichung (2) produziert werden, eine Molmasse von 500 bis 1400 g/mol haben und mit der allgemeinen Formel (5) H(OCH2CH2)ₙ - O - {CO - R2 - COO- [CH2CH2O]ₙ}ₘ H bezeichnet werden, wo R2 ein an der geraden Position ersetzter Benzolring mit der Molekularformel C6H4, a n>4 ist.

7. Ein Verfahren gemäß einem der vorstehenden beliebigen Ansprüche 1 bis 4, **gekennzeichnet dadurch, dass** Tenside mit plastifizierenden Eigenschaften in Übereinstimmung mit der Gleichung eine Molmasse zwischen 750 und 2350 g/mol haben und mit der allgemeinen Formel (6) R1 - COO - R2 - COO-{(CH2CH2O)n - CO - R2 - COO}m - [CH2CH2O]nH bezeichnet werden, wo R1 2-Ethylhexylalkylkette mit der Molekularformel C8H17, R2 ein an der geraden Position ersetzter Benzolring mit der Molekularformel C6H4, n>4 und m≥1 sind.

8. Ein Verfahren gemäß Anspruch 1, das den Veresterungsprozess unter Zusatz eines metallorganischen Katalysators und des 2-Ethylhexanols im Umesterungsprozess umfasst, **gekennzeichnet dadurch, dass** der Veresterungsprozess durch die Einführung des 2-Ethylhexanols und der Alkohole mit dem Kohlenstoffgehalt in der Moleküle C-C4 und der Säuren mit dem Kohlenstoffgehalt in der Moleküle C4-C6 im Gewichtsverhältnis der sauren Substrate von 25 bis 60 % sowie der Substrate, die eine Hydroxylgruppe von 40 bis 75 % massenhaft enthalten, durchgeführt wird, wo der 2-Ethylhexanol von 10 bis 80 % der Masse von allen Hydroxyl enthaltenden Substrate bildet, und andere Hydroxyl enthaltende Substrate von 20 bis 90 % massenhaft bilden, wobei der Inhalt des Reaktors bis zur Temperatur von 150 bis 180 °C unter absolutem Druck von 4. von 0 bis 66,7 kPa (30 bis 500 mmHg) angewärmt wird, und der metallorganische Katalysator in einer Menge von 0,02 bis 2% der Masse dosiert wird, wobei der Prozess zwecks der Erzielung von Parametern der Säurezahl unterhalb von 1 mgKOH/10b0g und der Viskosität im Bereich von 15 bis 1000 mPas bei der gleichzeitigen Abführung von Nebenprodukten in Form von Wasser und 2-Ethylhexanol geführt wird, wo anschließend der Prozess mit der Abkühlung des Produktes bis auf die Temperatur von 80 bis 120 °C und dem Ausschließen des Unterdruckes endet, und der Umesterungsprozess in Übereinstimmung mit folgenden Reaktionen nach den allgemeinen Gleichungen stattfindet:
(7) (n+1) OH-R3-OH + (n+1) COOH-R4-COOH + R1-OH -> OH-R3-COO-R4-COO-[R1-COO-R4-COO]n-R1 + (n+2)H2O,
(8) HOOC - R4 - COOH + HO-R3-OH + 2 R1-OH -> OH-R3-COO-R4-CO-O-R3-COO-R4-COO-R1 + 2H2O
(9) n HO-R3-OH + (n+1) HOOC-R4-COOH + (n+1) R1OH -> R1-COO-R4-COO-[R3-COO-R4-COO]n-R1 + (n+3) H2O
wo:
R1 - aliphatische Alkylkette des 2-Etylohexanols mit der Molekularformel C8H17 bedeutet;
R3 - aliphatische Alkylkette mit der Anzahl der Kohlenstoffatome 2-4 (C2-C4) bedeutet;
R4 - aliphatische Alkylkette mit der Anzahl der Kohlenstoffatome 4-6 (C4-C6) sowie n ≥ 1 bedeutet.

9. Ein Verfahren, beschrieben in Anspruch 8, **gekennzeichnet dadurch, dass** der Veresterungsprozess unter dem Druck von 6,7 bis 10,7 kPa (50 do 80 mmHg) stattfindet.

10. Ein Verfahren gemäß Anspruch 8 oder 9, **gekennzeichnet dadurch, dass** das Destillat der Nebenprodukte in Form von Wasser und 2-Ethylhexanol in den Empfänger, und anschließend in den Behälter weitergegeben wird, in dem die Separation der Phasen zwischen dem technologischen Wasser und 2-Ethylhexanol erfolgt, demgegenüber beträgt der Gehalt vom 2-Ethylhexanol im Nebenprodukt bis zu 70 % der Masse.

11. Ein Verfahren gemäß einem der vorstehenden beliebigen Ansprüche von 8 bis 10, **gekennzeichnet dadurch, dass** die nach der Formel (7) produzierten Plastifiziermittel eine Molmasse 450 bis 2000 g/mol haben und mit der allgemeinen Formel (10) OH-R3-COO-R4-COO-[R1-COO-R4-COO]n-R1 + (n+2)H2O bezeichnet werden, wo: R1 - aliphatische Alkylkette des 2-Etyloxenols, R3 - aliphatische Alkylkette mit einer gewissen Anzahl der Kohlenstoffatome 2-4 (C2-C4), R4 - aliphatische Alkylkette mit einer gewissen Anzahl der Kohlenstoffatome 4-6 (C4-C6) bedeutet; sowie n ≥ 1 ist,

12. Ein Verfahren gemäß einem beliebigen der vorstehenden Ansprüche von 8 bis 10, **gekennzeichnet dadurch, dass** die in Übereinstimmung mit der Gleichung (8) produzierten Plastifiziermittel eine Molmasse 550-650 g/mol haben und mit der allgemeinen Formel (11) OH-R3-COO-R4-CO-O-R3-COO-R4-COO-R1 bezeichnet werden, wo: R1 - eine aliphatische Alkylkette des 2-Ethylhexanols, R3 - eine aliphatische Alkylkette mit einer gewissen Anzahl der Kohlenstoffatome 2-4 (C2-C4), R4 - eine aliphatische Alkylkette mit einer gewissen Anzahl der Kohlenstoffatome 4-6 (C4-C6) bedeuten; sowie n ≥ 1 ist.

13. Ein Verfahren gemäß einem beliebigen der vorstehenden Ansprüche von 8 bis 10, **gekennzeichnet dadurch, dass** die in Übereinstimmung mit der Gleichung (9) produzierten Plastifiziermittel eine Molmasse zwischen 700 und 850 g/mol haben und mit der allgemeinen Formel (12) R1-COO-R4-COO-[R3-COO-R4-COO]n-R1 bezeichnet werden, wo: R1 - eine aliphatische Alkylkette des 2-Ethylohexanols, R3 - eine aliphatische Alkylkette mit einer gewissen Anzahl der Kohlenstoffatome 2-4 (C2-C4); R4 - eine aliphatische Alkylkette mit einer gewissen Anzahl der Kohlenstoffatome 4-6 (C4-C6) bedeuten, sowie n ≥ 1 ist.

14. Ein Verfahren gemäß einem beliebigen der vorstehenden Ansprüche von 1 bis 13, **gekennzeichnet dadurch, dass** die Dosierung des Katalysators in einer Temperatur erfolgt, die 1800C nicht überschreitet.

15. Ein Verfahren gemäß einem der beliebigen der vorstehenden Ansprüche von 1 bis 14, **gekennzeichnet dadurch, dass** Titan, Zirconium oder Zinnverbindungen als Katalysatoren eingesetzt werden.

16. Ein Verfahren gemäß Anspruch 15, **gekennzeichnet dadurch, dass** als Katalysatoren Tetra-n-butyltitanat, Tetra-n-butylzirkonat, Zinnoktanoat, Dibutylzinnoxid oder Dioctylzinnoxid verwendet werden sollen.

## Revendications

1. Le procédé d'obtention de tensioactifs et de plastifiants de haut poids moléculaire par transestérification en présence d'un catalyseur organométallique **caractérisé en ce que** la réaction de transestérification a lieu en introduisant dans le réacteur du téréphtalate de di(2-éthylhexyle) en quantités de 30 à 80 % en poids et de 20 à 70 % en poids de polyéthylène glycol de poids moléculaire de 200 à 600 g/mol, et **en ce que** le contenu du réacteur est chauffé avec agitation actionnée et apport d'azote de 50 à 200 l/min à 160 à 200 °C, ensuite un catalyseur organométallique liquide en quantité de 0,02 à 2 % en masse est introduit dans le milieu réactionnel, et le processus est réalisé afin d'obtenir les paramètres supposés conformément au bilan massique, en transférant simultanément au réacteur l'alcool 2-éthylhexylique obtenu, après quoi le processus est complété par un refroidissement rapide du produit prêt restant dans le réacteur à une température de 80 à 120 °C et par l'élimination de la pression négative, et le processus de transestérification est effectué conformément aux réactions suivantes des équations générales :
(1) R1 - COO - R2 - COO- R1 + H(OCH2CH2)nOH ↔ R1 - COO - R2 - COO-[CH2CH2O]ₙH + R1OH,
(2) m R1 - COO - R2 - COO- [CH2CH2O]ₙH + H(OCH2CH2)ₙOH ↔ H(OCH2CH2)ₙ - O -{CO - R2 - COO- [CH2CH2O]ₙ}ₘ H + m R1OH,
et
(3) H(OCH2CH2)n - O - {CO - R2 - COO- [CH2CH2O]n}m H + R1 - COO - R2-COO- R1 ↔ R1 - COO - R2 - COO-{(CH2CH2O)n - CO - R2 - COO}m-[CH2CH2O]nH + R10H,
où :
R1 - signifie une chaîne alkyle 2-éthylhexyle avec la formule moléculaire C8H17 ; R1OH - signifie l'alcool 2-éthylhexyle avec la formule moléculaire C8H180 ;
R2 est un anneau benzénique remplacé en position paire par la formule moléculaire C6H4 ; R1 - COO - R2 - COO - R1 est le téréphtalate de dioctyle ayant la formule moléculaire C24H38O4 ; H(OCH2CH2)nOH est le polyéthylène glycol, n>4 et m≥1.

2. Le procédé selon la revendication 1, **caractérisé en ce que** le processus de transestérification s'effectue à des pressions absolues de 4,0 à 66,7 kPa (30 à 500 mmHg), de préférence de 6,7 à 10,7 kPa (50 à 80 mmHg).

3. Le procédé selon la revendication 1 ou 2, **caractérisé en ce que** le processus de transestérification s'effectue à une vitesse d'agitation de 50 à 1000 tr/min.

4. Le procédé selon l'une quelconque des revendications d'1 à 3 **caractérisé en ce que** de l'alcool 2-éthylhexylique en quantité de 15 à 35 % en poids du produit principal comme sous-produit est produit au cours du processus de transestérification.

5. Le procédé selon l'une quelconque des revendications d'1 à 4 **caractérisé en ce que** les agents tensioactifs ayant des propriétés plastifiantes produits selon l'équation (1) ont un poids moléculaire compris entre 450 et 900 g/mol et sont déterminés par la formule générale (4) R1 - COO - R2 - COO - [CH2CH2O]nH , dans laquelle R1 est une chaîne 2-éthylhexyle alkyle de formule moléculaire C8H17, R2 est un anneau benzénique remplacé en position paire ayant la formule moléculaire C6H4 et n>4.

6. Le procédé selon l'une quelconque des revendications d'1 à 4 **caractérisé en ce que** les agents tensioactifs ayant des propriétés plastifiantes produits selon l'équation (2) ont un poids moléculaire compris entre 500 et 1400 g/mol et sont déterminés par la formule générale (5) H(OCH2CH2)ₙ - O - {CO - R2 - COO- [CH2CH2O]ₙ}ₘ H, où R2 est un anneau benzénique remplacé en position paire ayant la formule moléculaire C6H4 et n>4.

7. Le procédé selon l'une quelconque des revendications d'1 à 4 **caractérisé en ce que** les agents tensioactifs ayant des propriétés plastifiantes produits selon l'équation ont un poids moléculaire compris entre 750 et 2350 g/mol et sont déterminés par la formule générale (6) R1 - COO - R2 - COO-{(CH2CH2O)n - CO - R2 - COO}m - [CH2CH2O]nH, où R1 est une chaîne 2-éthylhexyle alkyle de formule moléculaire C8H17, R2 est un anneau benzénique remplacé en position paire ayant la formule moléculaire C6H4, n>4 et m≥1.

8. Le procédé selon la revendication 1, comprenant en outre une estérification en présence d'un catalyseur organométallique et d'alcool 2-éthylhexylique résultant du procédé de transestérification, **caractérisé en ce que** l'estérification est effectuée en introduisant de l'alcool 2-éthylhexylique et les alcools ayant une teneur en carbone dans la molécule C-C4 et les acides ayant une teneur en carbone dans la molécule C4-C6 dans un rapport de 25 à 60 % en poids de substrats acides et de substrats contenant un groupe hydroxyle de 40 à 75 % en poids, où l'alcool 2-éthylhexylique représente 10 à 80 % en poids de tous les substrats contenant de l'hydroxyle, et les autres substrats contenant de l'hydroxyle représentent 20 à 90 % en poids, puis le contenu du réacteur est chauffé à 150 à 180 °C à une pression absolue de 4. de 0 à 66,7 kPa (30 à 500 mmHg) et un catalyseur organométallique est dosé en une quantité de 0,02 à 2 % en poids, après quoi le processus est réalisé afin d'obtenir des paramètres d'indice d'acide inférieurs à 1 mgKOH/100g et une viscosité dans la gamme de 15 à 1000 mPas, avec évacuation simultanée des sous-produits sous forme d'eau et d'alcool 2-éthylhexylique, après quoi le procédé est complété par le refroidissement du produit à une température de 80 à 120 °C et l'élimination de la pression négative, et l'estérification est réalisée selon des réactions avec des équations générales :
(7) (n+1) OH-R3-OH + (n+1) COOH-R4-COOH + R1-OH -> OH-R3-COO-R4-COO-[R1-COO-R4-COO]n-R1 + (n+2)H2O,
(8) HOOC - R4 - COOH + HO-R3-OH + 2 R1-OH -> OH-R3-COO-R4-CO-O-R3-COO-R4-COO-R1 + 2H2O
(9) n HO-R3-OH + (n+1) HOOC-R4-COOH + (n+1) R1OH -> R1-COO-R4-COO-[R3-COO-R4-COO]n-R1 + (n+3) H2O
où :
R1 - signifie la chaîne alkyle de l'alcool 2-éthylhexylique de formule moléculaire C8H17 ;
R3 - signifie une chaîne alkyle aliphatique avec un nombre de carbones de 2 à 4 (C2-C4) ;
R4 signifie une chaîne alkyle aliphatique avec un nombre de carbones de 4 à 6 (C4-C6) et n ≥ 1.

9. Le procédé décrit dans la revendication 8, **caractérisé en ce que** l'estérification s'effectue sous une pression de 6,7 à 10,7 kPa (50 à 80 mmHg).

10. Le procédé selon la revendication 8 ou 9, **caractérisé en ce que** le distillat sous-produit sous forme d'eau et d'alcool 2-éthylhexylique est transféré dans un récepteur puis dans un récipient dans lequel il y a une séparation des phases entre l'eau de traitement et l'alcool 2-éthylhexylique, alors que la teneur en alcool 2-éthylhexylique dans le sous-produit peut atteindre 70 % en poids.

11. Le procédé selon l'une quelconque des revendications de 8 à 10, **caractérisé en ce que** les plastifiants fabriqués selon la formule (7) ont un poids moléculaire de 450 à 2000 g/mol et sont déterminés à l'aide de la formule générale OH-R3-COO-R4-COO-[R1-COO-R4-COO]n-R1 + (n+2)H2O où : R1 - une chaîne alkyle d'alcool 2-éthylhexylique, R3 - une chaîne alkyle aliphatique avec un certain nombre d'atomes de carbone de 2 à 4 (C2-C4), R4 - une chaîne alkyle aliphatique avec un certain nombre d'atomes de carbone de 4 à 6 (C4-C6) ; et n ≥ 1,

12. Le procédé selon l'une quelconque des revendications de 8 à 10 **caractérisé en ce que** les plastifiants produits selon l'équation (8) ont un poids moléculaire de 550-650 g/mol et sont déterminés par la formule générale (11) OH-R3-COO-R4-CO-O-R3-COO-R4-COO-R1, où : R1 - chaîne alkyle d'alcool 2-éthylhexylique, R3 - chaîne alkyle aliphatique avec un certain nombre d'atomes de carbone de 2 à 4 (C2-C4), R4 - chaîne alkyle aliphatique avec un certain nombre d'atomes de carbone de 4 à 6 (C4-C6) ; et n ≥ 1.

13. Le procédé selon l'une quelconque des revendications de 8 à 10 **caractérisé en ce que** les plastifiants produits selon l'équation (9) ont un poids moléculaire compris entre 700 et 850 g/mol et sont déterminés par la formule générale (12) R1-COO-R4-COO-[R3-COO-R4-COO]n-R1, où : R1 - chaîne alkyle de l'alcool 2-éthylhexylique, R3 - chaîne alkyle aliphatique avec un certain nombre d'atomes de carbone de 2 à 4 (C2-C4) ; R4 - chaîne alkyle aliphatique avec un certain nombre d'atomes de carbone de 4 à 6 (C4-C6), et n ≥ 1.

14. Le procédé selon l'une quelconque des revendications d'1 à 13, caractérisé en ce le dosage du catalyseur se fait à une température ne dépassant pas 180°C.

15. Le procédé selon l'une quelconque des revendications d'1 à 14 **caractérisé en ce que** des composés de titane, de zirconium ou d'étain soient utilisés comme catalyseurs.

16. Le procédé selon la revendication 15 **caractérisé en ce que** les catalyseurs doivent être le titanate de tétra-n-butyle, le zirconate de tétra-n-butyle, l'octanoate d'étain, l'oxyde de dibutylétain ou l'oxyde de dioctylétain.
